# EUROPEAN PATENT SPECIFICATION

(11) **EP 1 325 146 B1**
(45) Date of publication and mention of the grant of the patent: **14.12.2005**
(21) Application number: 01978420.6
(22) Date of filing: 12.10.2001
(51) Int. Cl.: C12P 17/10

(54) **PROCESS FOR PREPARING OPTICALLY ACTIVE 4-HYDROXY-2-PYRROLIDINONE AND N-SUBSTITUTED 4-HYDROXY-2-PYRROLIDINONES BY ENZYMATIC HYDROXYLATION**
VERFAHREN ZUR HERSTELLUNG OPTISCH AKTIVER 4-HYDROXY-2-PYRROLIDINONE UND N-SUBSTITUIERTEN 4-HYDROXY-2-PYRROLIDINONEN DURCH ENZYMATISCHE HYDROXYLIERUNG
PROCEDE DE PREPARATION DE 4-HYDROXY-2-PYRROLIDINONE ET DE 4-HYDROXY-2-PYRROLIDINONES N-SUBSTITUEES OPTIQUEMENT ACTIVES PAR HYDROXYLATION ENZYMATIQUE

(30) Priority: 13.10.2000 EP 00203579
(43) Date of publication of application: 09.07.2003
(73) Proprietor: Eidgenössiche Technische Hochschule Zürich, 8093 Zürich (CH)
(72) Inventor: LI, Zhi, CH-8902 Urdorf (CH); CHANG, Dongliang, CH-8048 Zürich (CH); WITHOLT, Bernard, CH-8032 Zürich (CH)
(74) Representative: Prins, Adrianus Willem
(86) International application number: PCT/EP2001/011931
(87) International publication number: WO 2002/031174

(56) References cited:
- EP-A- 1 002 871
- SRAIRI, DRISS ET AL: "Hydroxylation microbiologiques de pyrrolidinones-2 (note de laboratoire)" BULL. SOC. CHIM. FR. (1987), vol. 2, pages 297-301, XP000982887 cited in the application
- CHANG, DONGLIANG ET AL: "Preparation of (S)-N-Substituted 4-Hydroxypyrrolidin-2-ones by Regio- and Stereoselective Hydroxylation with Sphingomonas sp. HXN -200" ORG. LETT. (2000), 2(24), 3949-3952 , XP002162512

## Description

### Field of the invention:

The present invention relates to a process for preparing optically active 4-hydroxy-2-pyrrolidinone and N-substituted 4-hydroxy-2-pyrrolidinones, which are useful as intermediates for the preparation of several pharmaceutical products, wherein an oxygen atom is inserted regio- and stereoselectively into the corresponding 2-pyrrolidinones by use of biocatalysts.

### Description of the Prior Art:

Optically active 4-hydroxy-2-pyrrolidinone and its N-substituted derivatives are useful intermediates for the syntheses of several pharmaceuticals.

In practice it is often advantageous to use optically active 4-hydroxy-2-pyrrolidinone in its N-protected form.

It is known that (S)- and (R)-4-hydroxy-2-pyrrolidinone can be prepared from the corresponding (R)-4-chloro-3-hydroxy-butanoate [Pellegata, R., et al, Tetrahedron, 1985, 41, 5607] or (S)-4-tosyl-3-hydroxy-butanoate [Seki, M., et al, Synthesis, 1999, 5, 745] via cyclization with ammonia. They can also be prepared by direct, cyclization of (S)- or (R)-4-amino-3-hydroxy-butanoate [Kobayashi, S., et al, Synlett. 1999, S1, 909; EP 0947505 A2 (6 Oct. 1999); EP 0844242 A1 (27 May 1998); JP 9031058 A2 (4 Feb. 1997)] and (S)- or (R)-4-azido-3-hydroxy-butanoate [JP 8119935 (14 May. 1996); EP 0844242 A1 (27 May 1998); WO 9636603 (21 Nov. 1996)], respectively. (S)- and (R)-N-substituted 4-hydroxy-2-pyrrolidinones can be prepared by cyclization of (R) or (S)-4-halo-3-hydroxy-butanoate with alkyl or aralkyl amine [JP 7324071 A2 (12 Dec. 1995); JP 1045360 A2 (17 Feb. 1989)] and by direct cyclization of (S)-or (R)-N-substituted 4-amino-3-hydroxy-butyric acid or ester [EP 0787718 A1 (6 Aug. 1997); Orena, M., et al, J. Chem. Res. (S), 1990, 376; Srairi, D. etal, Bull. Soc. Chim. Fr., 1987, 2, 297-301], (S)- or (R)-N-substituted 4-halo-3-hydroxy-butanoic amide [JP 8319271 A2 (3 Dec. 1996)], and (S)-4-mesyl-3-benzyloxybutanoic acid benzyl amide [Inagaki, S., et al, Enantiomer, 1998, 3, 187], respectively. However, all such processes have common drawbacks: the (S)- and (R)- precursors are not easily available and have to be prepared in multi-step reactions; the cyclization processes are not efficient and require special agents.

One process for the preparation of (S)-N-benzyl-4-hydroxy-2-pyrrolidinones involves the multi-step reduction of (S)-N-benzyl-4-hydroxypyrrolidin-2,5-dione [Huang, P.Q., et al, Tetrahedron: asymmetry, 1999, 10, 3309]. This synthesis is not practical for preparation on larger scales and requires special reagents.

(R)-N-substituted 4-hydroxy-2-pyrrolidinone can be prepared in multi-steps from the expensive (2S, 4R)-4-hydroxyproline [Haeusler, J. Monatsh. Chem. 1987, 118, 865]. This method, however, is not suitable for industrial production.

(R)-N-substituted 4-hydroxy-2-pyrrolidinone can also be prepared by photolysis of 5-phenylmethyloxy-2-(1'-phenylethyl)-1-oxa-2-azaspiro[2,3]-hexane [Aube, J., et al, Syn. Commun. 1991; 21, 693]. The yield is low (16%).

Resolution of racemic 4-hydroxy-2-pyrrolidinones with a stereoselective esterase is known [JP 10165195 A2 (23 Jun. 1998)]. However, the yield is low, and the racemate needs to be prepared in multi-steps.

A more direct and economic method to prepare optically active 4-hydroxy-2-pyrrolidinone and its N-substituted derivatives could be regio- and stereoselective insertion of one oxygen atom into the corresponding 2-pyrrolidinones, which are easily available. However, this reaction is not possible with classical chemical synthesis.

In the only one report about enzymatic hydroxylation of 2-pyrrolidinones [Srairi, D., et al, Bull. Soc. Chim. Fr. 1987, 297], Beauveria sulfurescens (ATCC 7159), a well-known fungus for biohydroxylation, was used to catalyse hydroxylation of N-benzoyl-2-pyrrohdinone and N-phenylacetyl-2-pyrrolidinone. However, the regio- and stereoselectivity were very poor. Several products were formed in each case, and N-benzoyl-4-hydroxy-2-pyrrolidinone and N-phenylacetyl-4-hydroxy-2-pyrrolidinone were formed in only 21% and 5%, respectively, with low enantiomeric excess (e.e.). Furthermore, the concentration of product is too low, the reaction is too slow, and the separation of the product from the biotransformation mixture is difficult. This process is not suitable for large-scale preparation of the product.

### Description of the invention

Here we have developed a process for the preparation of optically active 4-hydroxy-2-pyrrolidinone or an N-substituted 4-hydroxy-2-pyrrolidinone by hydroxylation of a corresponding non-hydroxylated 2-pyrrolidinone or N-substituted 2-pyrrolidinone using as a biocatalyst alkane-degrading bacteria having hydroxylation activity to insert an oxygen atom regio- and stereoselectively into said corresponding non-hydroxylated 2-pyrrolidinone or N-substituted 2-pyrrolidinone.

By use of a miniaturised microtiter plate based screening system, we found that many bacteria catalyse the regio- and stereoselective insertion of an oxygen atom into 2-pyrrolidinone or N-substituted 2-pyrrolidinones giving the corresponding optically active 4-hydroxy-2-pyrrolidinone or N-substituted 4-hydroxy-2-pyrrolidinones.

In a typical screening procedure demonstrated in example 1, 96 alkane-degrading strains were grown on the vapour of a mixture of n-hexane/n-octane/n-decane/n-dodecane/n-tetradecane (1:1:1:1:1) in agar-based mineral growth medium on a microtiter plate for 3-6 days. The cells from each well were harvested and resuspended in 150 µl of 50 mM phosphate buffer (pH = 7.2) containing 1-2% glucose in the wells of a microtiter plate. N-Benzyl-2-pyrrolidinone (0.1-1.0 mM) was added, and the mixture was shaken at 200 rpm and at 25°C for 2 h. The formation of N-benzyl-4-hydroxy-2-pyrrolidinone was determined for each of the wells by high performance liquid chromatography (HPLC) coupled with MS detection.

It has been found that many bacteria that degrade alkanes are able to catalyse regio- and stereoselectively hydroxylation of 2-pyrrolidinone or N-substituted 2-pyrrolidinones giving the corresponding optically active 4-hydroxy-2-pyrrolidinone or N-substituted 4-hydroxy-2-pyrrolidinones. Preferred biocatalysts are bacteria degrading alkanes containing 4 to 20 C atoms, such as bacteria that degrade n-hexane, n-heptane, n-octane, n-nonane, n-decane, n-undecane, n-dodecane, n-tridecane, or n-tetradecane. Examples of such biocatalysts are the isolates Sphingomonas sp. HXN-200, HXN-100, and HXN-1500.

It has also been found that many bacteria that degrade cyclic hydrocarbons are able to catalyse regio- and stereoselectively hydroxylation of 2-pyrrolidinone or N-substituted 2-pyrrolidinones giving the corresponding optically active 4-hydroxy-2-pyrrolidinone or N-substituted 4-hydroxy-2-pyrrolidinones. Preferred biocatalysts are the bacteria degrading cyclic compounds containing 4 to 20 C atoms, such as bacteria that degrade cyclopentane, cyclohexane, cycloheptane, cyclooctane, cyclononane, cyclodecane, cycloundecane, cyclododecane, cyclotridecane, or cyclotetradecane.

It has been found that many bacteria having alkane hydroxylase(s) are able to catalyse regio- and stereoselectively hydroxylation of 2-pyrrolidinone or N-substituted 2-pyrrolidinones giving the corresponding optically active 4-hydroxy-2-pyrrolidinone or N-substituted 4-hydroxy-2-pyrrolidinones.

It has been found that many bacteria that are able to oxidize hydrocarbons are able to catalyse regio- and stereoselectively hydroxylation of 2-pyrrolidinone or N-substituted 2-pyrrolidinones giving the corresponding optically active 4-hydroxy-2-pyrrolidinone or N-substituted 4-hydroxy-2-pyrrolidinones.

The biotransformation can be performed with resting cells, with growing cells, or with both as biocatalysts.

The biotransformation can be also performed with crude cell extracts as biocatalysts.

The biocatalysts can be immobilized on or in a water-insoluble carrier or support system.

The biotransformation can be carried out in aqueous medium. It can also be performed in multiphase media possibly containing two or more of the following: a solid phase, an aqueous phase, an organic phase, or a gasiform phase. Organic phase can comprises one or more of the following: alkanes with 5 or more C atoms; dialkyl ethers with 4 or more C atoms; carboxylic esters with 3 or more C atoms; aromatic or heteroaromatic hydrocarbons with 6 or more C atoms; or alkyl aromatic or alkyl heteroaromatic hydrocarbons with 7 or more C atoms. An example of a suitable organic solvent is hexadecane.

The enzymatic hydroxylations can be carried out, although this is not a critical parameter, at a temperature of 5-50°C, preferably at 20-40°C. The pressure can vary within wide limits. In practice the biotransformation is performed at atmospheric pressure. The pH of the reaction medium can be between 4 and 10, preferably between 6 and 8.

The product can be separated by chromatographic techniques with an inorganic, organic, or synthetic adsorbent used as a support. The suitable adsorbents are, for instance, aluminium oxide and silica gel. The product can be also isolated by membrane filtration.

The product can be also separated by means of extraction, wherein the substrate is first recovered from the reaction mixture by extraction with less polar solvent, the remaining reaction mixture is adjusted to pH = 8-12, and the product is extracted out with more polar solvent. The suitable extraction agent used is selected from the group consisting of: alkanes with 5 or more C atoms; dialkyl ethers with 4 or more C atoms; chlorine-containing alkanes with 3 or fewer C atoms; carboxylic esters with 3 or more C atoms; aromatic or heteroaromatic hydrocarbons with 6 or more C atoms; alkyl aromatic or alkyl heteroaromatic hydrocarbons with 7 or more C atoms. Examples of particularly suitable extraction agents are hexane and ethyl acetate, as apolar and polar solvent, respectively.

It has been found that the product can be also separated by means of solid phase extraction.

Sphingomonas sp. HXN-200 (isolated by Plaggemeier, Th.; Schmid, A.;' Engesser, K. at the University of Stuttgart; in the strain collection of the Institute of Biotechnology, ETH Zurich) has been found to be an excellent biocatalyst for the regio- and stereoselective hydroxylation of 2-pyrrolidinone or N-substituted 2-pyrrolidinones giving the corresponding optically , active 4-hydroxy-2-pyrrolidinone or N-substituted 4-hydroxy-2-pyrrolidinones. Examples of substituted 4-hydroxy-2-pyrrolidinones are N-benzyl-, N-tert-butoxycarbonyl-, N-benzyloxycarbonyl-, N-phenoxycarbonyl-, N-benzoyl-, and N-tosyl-4-hydroxy-2-pyrrolidinones.

The cells of Sphingomonas sp. HXN-200 can be prepared in large scale by growing in E2 medium (Lageveen, R. G. et al, Appl. Environ. Microbiol. 1988, 54, 2924) either with n-octane as carbon source or with glucose as carbon source followed by induction of the alkane oxidation system with dicyclopropyl ketone (DCPK) or n-octane. The cells can be stored at -80°C for several months and can be used and handled like normal chemical reagents in a bioconversion with resting cells.

A typical procedure for hydroxylation with resting cells is demonstrated in example 2. N-benzyl-2-pyrrolidinone (2-5 mM) was added to 10 ml of cell suspension (4.0 g/L) of Sphingomonas sp. HXN-200 in 50 mM K-phosphate buffer (pH = 8.0) containing glucose (0 or 2%), and the mixture was shaken at 30°C for 5 h. The reaction was followed by HPLC analysis: samples (0.10 ml) were taken out directly from the reaction mixture at different times, the cells were removed by centrifugation, and the supernatants were analysed by analytical HPLC [column: Hypersil BDS-C18 (5 µm, 125 mm x 4 mm), eluent: acetonitrile/10mM K-phosphate buffer (pH 7.0) 2:8, flow: 1.0 ml/min., and UV detection: 210, 225, and 254 nm]. Retention time of N-benzyl-4-hydroxy-2-pyrrolidinone: 2.7 min.; retention time of N-benzyl 2-pyrrolidinone: 8.1 min.

A procedure for standard work-up was established: the cells were removed by centrifugation, the supernatants were adjusted to pH = 8-12 by the addition of KOH followed by extraction with ethyl acetate. The organic phase was dried over MgSO₄, filtered, and the solvent evaporated.

The product was purified by column chromatography on silica gel (R_{f} = 0.14, ethyl acetate/CHCl₃ 9/1). The structure was identified by ¹H- and ¹³C-NMR and MS spectra.

As shown in table 2, hydroxylation of N-benzyl-2-pyrrolidinone with resting cells (4.0 g/L) of Sphingomonas sp. HXN-200 gave N-benzyl-4-hydroxy-2-pyrrolidinone in high conversion in the presence of 2% glucose. 70 % and 47% of N-benzyl-4-hydroxy-2-pyrrolidinone were formed at 5 h for hydroxylation of 2 mM and 5 mM of N-benzyl-2-pyrrolidinone, respectively.

Hydroxylation of N-tert-butoxycarbonyl 2-pyrrolidinone with resting cells of Sphingomonas sp. HXN-200 was demonstrated in example 3. Bioconversion was performed with 2-18 mM of N-tert-butoxycarbonyl-pyrrolidinone in 10 ml of cell suspension (4 g/L) of Sphingomonas sp. HXN-200 in 50 mM K-phosphate buffer (pH = 8.0) in the presence of glucose (2%) at 30°C for 5 h. The reaction was followed by HPLC analysis, samples were prepared by taking out 0.10 ml of the reaction mixture at different times and removal of cells by centrifugation.

Retention time of N-tert-butoxycarbonyl-4-hydroxy 2-pyrrolidinone: 2.7 min.; retention time of N-tert-butoxycarbonyl-2-pyrrolidinone: 6.7 min. [column: Hypersil BDS-C18 (5 µm, 125 mm x 4 mm), eluent: acetonitrile/10mM K-phosphate buffer (pH 7.0) 2:8, flow: 1.0 ml/min., and UV detection: 210, 225, and 254 nm].

As shown in table 3, hydroxylation of N-tert-butoxycarbonyl-2-pyrrolidinone gave high conversion to N-tert-butoxycarbonyl-4-hydroxy-2-pyrrolidinone. 80% of product was formed for the hydroxylation of 2 mM of substrate for 5 h. The hydroxylation is not very much dependent on the starting concentration of the substrate. Hydroxylation of 18 mM of N-tert-butoxycarbonyl-2-pyrrolidinone gave 48% of N-tert-butoxycarbonyl-4-hydroxy-2-pyrrolidinone. The highest activity was achieved for the hydroxylation of 14 mM of substrate: 10.7 U/g CDW. In this case, 63% of conversion was reached in 5 h.

It has been found that preparative scale hydroxylation can be easily carried out in a shaking flask. Example 4 demonstrated the hydroxylation of N-benzyl-2-pyrrolidinone with resting cells of Sphingomonas sp. HXN-200 in 50 ml of 50 mM K-phosphate buffer (pH = 8.0) containing glucose (2%), in a 500 ml shaking flask. Biotransformation was performed at 200 rpm and 30°C for 5 h. Hydroxylation of 3 mM of N-benzyl-2-pyrrolidinones with 4.0 g/L of cell suspension formed 66% (1.98 mM) of N-benzyl-4-hydroxy-2-pyrrolidinone. Standard work-up and column chromatography on silica gel (R_{f} = 0.14, ethyl acetate/CHCl₃ 9:1) afforded 55% (0.32 g/L) of pure product as white powder.

It has been found that the concentration of product can be increased by use of higher concentration of substrate and higher cell density. As shown in table 4, hydroxylation of 6.0 mM of N-benzyl-2-pyrrolidinone with 8.0 g/L of cell suspension formed 51 % (3.06 mM) of product at 5 h. Standard work-up and column chromatography afforded 42% (0.48 g/L) of pure product.

Hydroxylation of 6.0 mM of N-benzyl-2-pyrrolidinone with 10.0 g/L of cell suspension gave 75 % (4.5 mM) of product at 5h. 68% (0.78 g/L) of pure product was isolated.

The e.e: of N-benzyl-4-hydroxy-2-pyrrolidinone was measured by analytical HPLC with a chiral column [Chiralpak AS (Daicel), 250 mm x 4.6 mm; eluent: hexane/isopropanol (4:1); flow rate: 1.0 ml/min.; detection wavelength: 210nm; retention times: 20.3 min. for the (S)-form and 30.5 min. for the (R)-form].

It has been found that the e.e. of N-benzyl-4-hydroxy-2-pyrrolidinone obtained from biohydroxylation is >99.9% e.e. (S).

Example 5 demonstrated the preparation of N-tert-butoxycarbonyl-4-hydroxy-2-pyrrolidinone in a 500 ml shaking flask. Hydroxylation of N-tert-butoxycarbonyl-2-pyrrolidinone was performed with resting cells of Sphingomonas sp. HXN-200 in 50 ml of 50 mM K-phosphate buffer (pH = 8.0) containing glucose (2%) at 200 rpm and 30°C for 5 h. Hydroxylation of 14 mM of N-tert-butoxycarbonyl-2-pyrrolidinone with 4.0 g/L of cell suspension formed 48% (6.72 mM) of N-tert-butoxycarbonyl-4-hydroxy-2-pyrrolidinone. Standard work-up and column chromatography on silica gel (R_{f} = 0.28, ethyl acetate) afforded 39% (1.10 g/L) of pure product as white powder.

It has been found that the concentration of N-tert-butoxycarbonyl-4-hydroxy-2-pyrrolidinone can be increased by use of higher cell density. Hydroxylation of 14 mM of N-tert-butoxycarbonyl-2-pyrrolidinone with 8.0 g/L of cell suspension for 5 h resulted in 66 % (9.24 mM) of the desired product. Standard work-up and column chromatography on silica gel afforded 46% (1.29 g/L) of pure product as white powder.

It has also been found that the concentration of N-tert-butoxycarbonyl-4-hydroxy-2-pyrrolidinone can be further increased by use of even higher concentrations of substrate and cells. Hydroxylation of 20 mM of N-tert-butoxycarbonyl-2-pyrrolidinone with 10.0 g/L of cell suspension for 5 h formed 49 % (9.8 mM) of N-tert-butoxycarbonyl-4-hydroxy-2-pyrrolidinone. The pure product was isolated in 42% yield (1:69 g/L).

The e.e. of N-tert-butoxycarbonyl-4-hydroxy-2-pyrrolidinone -was determined by analytical HPLC with a chiral column [Chiralcel OB-H (Daicel), 250 mm x 4.6 mm; eluent: hexane/isopropanol (7:1); flow rate: 0.5 ml/min.; detection wavelength: 210 nm; retention times: 17.9 min. for the (R)-form and 22.6 min. for the (S)-form]. N-tert-Butoxycarbonyl-4-hydroxy-2-pyrrolidinone obtained by biohydroxylation has 90-92% e.e. (S).

It has been found that the e.e. of N-tert-butoxycarbonyl-4-hydroxy-2-pyrrolidinone can be easily increased from 92% to 99.9% (S) in 82% yield by simple crystallization from n-hexane/ethyl acetate (2/1).

It has been found that the preparation of 4-hydroxy-2-pyrrolidinones can be easily performed in a bioreactor. Example 6 demonstrates the production of N-benzyl 4-hydroxy-2-pyrrolidinone on 1 L scale in a bioreactor. Hydroxylation of N-benzyl 2-pyrrolidinone (10.0 mM) with cell suspension (10.0 g/L) of Sphingomonas sp. HXN-200 in 1 L of 50 mM K-phosphate buffer (pH 8.0) containing glucose (2%) for 5 h formed 80% of N-benzyl-4-hydroxy-2-pyrrolidinone. Standard work-up and column chromatography on silica gel gave 70% (1.408 g) of pure (S)-N-benzyl-4-hydroxy-2-pyrrolidinone as white powder in >99.9% e.e..

In example 7, hydroxylation of N-tert-butoxycarbonyl-2-pyrrolidinone (14 mM) was carried out with cell suspension of Sphingomonas sp. HXN-200 of 10.2 g/L in 1 L of 50 mM K-phosphate buffer (pH 8.0) containing glucose (2%) in a bioreactor. 85% conversion to N-tert-butoxycarbonyl-4-hydroxy-2-pyrrolidinone was achieved in 5 h. Standard work-up and column chromatography afforded 2.008 g (75%) of pure N-tert-butoxycarbonyl-4-hydroxy-2-pyrrolidinone as white powder in 90% e.e. (S).

Biohydroxylation with growing cells as biocatalyst was demonstrated in example 8. Hydroxylation of N-benzyl-2-pyrrolidinone was performed with growing cells of Sphingomonas sp. HXN-200 on 1 L scale. The cells were grown in E2 medium first on glucose and then on n-octane to a cell density of 6.2 g/L. N-benzyl-2-pyrrolidinone (4.0 mM) was added, and the mixture was stirred at 1536 rpm and at 30°C with air introduction at 2 L/min. During bioconversion, n-octane vapour was still introduced and the cells still grew. Additional substrate was added at 1 h (2.0 mM), 2 h (2.0 mM), and 3 h (2.0 mM). The biotransformation was stopped at 4 h with 84 % conversion to N-benzyl 4-hydroxy-2-pyrrolidinone. Standard work-up and column chromatography afforded 76% (1.529 g) of pure product as white powder in >99.9% e.e. (S).

Biohydroxylation with crude cell extracts as biocatalyst -was demonstrated in example 9. Cells of Sphingomonas sp. HXN-200 were suspended in 10 ml of Tris-HCl buffer (pH = 7.5) to a density of 20 g/L. After passage through the French press three times, the cell debris and membranes were removed by centrifugation at 45, 000 rpm for 45 min. giving soluble cell-free extracts containing no membrane proteins. To this crude cell-free extracts was added NADH (2.0 mM) and N-benzyl piperidine (2.0 mM). The mixture was shaken at 200 rpm and at 30°C for 1 h afforded 80% of (S)-N-benzyl-4-hydroxy-2-pyrrolidinone in >99.9%.

The specific examples given herein are intended merely as an illustration of the invention and should not be construed as a restriction of the scope of the invention.

N-substituted 4-hydroxy-2-pyrrolidinone obtained by this process can be easily converted into 4-hydroxy-2-pyrrolidinone by deprotection.

In a summary, the invention here provides a useful method for the preparation of optically active 4-hydroxy-2-pyrrolidinones and N-substituted 4-hydroxy-2-pyrrolidinone.

### Examples

### Example 1: Screening of biocatalyst for hydroxylation of N-benzyl-2-pyrrolidinone giving N-benzyl-4-hydroxy-2-pyrrolidinone

Alkane-degrading strains were grown on the vapour of a mixture of n-hexane/n-octane/n-decane/n-dodecane/n-tetradecane (1:1:1:1:1) in agar-based Evans medium in the wells of a microtiter plate for 3-6 days. The cells from each well were harvested and resuspended in 150 µl of 50 mM phosphate buffer (pH = 7.2) containing 100 mM glucose in the wells of a microtiter plate. N-benzyl-2-pyrrolidinone was added to a final concentration of 150 µM. The mixture was shaken at 200 rpm and at 25°C for 2 h. Cells were removed by centrifugation of the microtiter plate, and the supernatants were analysed for the formation of N-benzyl-4-hydroxy-2-pyrrolidinone by HPLC-MS.

Conditions for HPLC-MS analysis: Nucleosil 100-5 C18 pre-column; eluent: A/B 94/6 form 0 to 0.8 min., gradient to A/B 65/35 till 1.10 min., A/B 65/35 from 1.10 to 3.5 min. (A: acetonitrile/10 mM K-phosphate buffer (pH 7.0), 1/9, B: acetonitrile); flow at 0.5 ml/min.; MS detection at 176 and 192; retention time of N-benzyl-4-hydroxy-2-pyrrolidinone: 0.7 min.; retention time of N-benzyl-2-pyrrolidinone: 2.0 min. The results are summarized in table 1.

**Table 1:**

| Hydroxylation of N-benzyl-2-pyrrolidinone giving N-benzyl-4-hydroxy-2-pyrrolidinone with several alkane-degrading bacteria | | |
|---|---|---|
| Entry | Strains¹ | Relative activity² |
| 1 | Sphingomonas sp. HXN-200 | 1 |
| 2 | HXN-100 | 4.0 |
| 3 | HXN-1500 | 0.5 |

| | | |
|---|---|---|
| ¹ Strains are isolated by use of n-hexane or n-octane as carbon source; all strains are in the strain collection of the Institute of Biotechnology, ETH Zurich. | | |
| ²Activity was compared with that of Sphingomonas sp. HXN-200. | | |

### Example 2: Hydroxylation of N-benzyl-2-pyrrolidinone giving N-benzyl-4-hydroxy-2-pyrrolidinone with resting cells of Sphingomonas sp. HXN-200

Sphingomonas sp. HXN-200 (isolated by Plaggemeier, Th.; Schmid, A.; Engesser, K. at University of Stuttgart; in the strain collection of the Institute of Biotechnology, ETH Zurich) was inoculated in 2 L of E2 medium with the vapour of n-octane as carbon source and grown at 30°C, the cells were harvested at a cell density of 2-10 g/L and stored at -80°C.

N-Benzyl-2-pyrrolidinone (2-5 mM) was added to 10 ml cell suspension (4.0 g/L) of HXN-200 in 50 mM K-phosphate buffer (pH 8.0) containing glucose (0 or 2%), and the mixture was shaken at 30°C. for 5 h. The reaction was followed by analytical HPLC: samples were taken out directly from the reaction mixture at different times, the cells were removed by centrifugation, and the supernatants were analysed. The results are listed in table 2.

**Table 2:**

| Hydroxylation of N-benzyl-2-pyrrolidinone giving N-benzyl-4-hydroxy-2-pyrrolidinone with resting cells (4 g/L) of Sphingomonas sp. HXN-200 | | | | | | | | |
|---|---|---|---|---|---|---|---|---|
| Entry | Substr. (mM) | Glucose (%) | Activity¹ (U/g CDW) | Conversion (%) | | | | |
| | | | | 0.5 h | 1 h | 2 h | 3 h | 5 h |
| 1 | 2 | 0 | 2.6 | 15 | 19 | 22 | 22 | 23 |
| 2 | 2 | 2 | 4.4 | 26 | 41 | 62 | 69 | 70 |
| 3 | 3 | 2 | 4.6 | 18 | 29 | 49 | 58 | 65 |
| 4 | 4 | 2 | 4.1 | 12 | 19 | 36 | 47 | 57 |
| 5 | 5 | 0 | 3.0 | 7 | 8 | 9 | 10 | 10 |
| 6 | 5 | 2 | 4.3 | 10 | 14 | 24 | 36 | 47 |

| | | | | | | | | |
|---|---|---|---|---|---|---|---|---|
| ¹Activity was determined over the first 30 min. | | | | | | | | |

Condition of analytical HPLC: column: Hypersil BDS-C18 (5 µm), 125 mm x 4 mm; eluent: acetonitrile/10mM K-phosphate buffer (pH 7.0) 2:8; flow rate: 1 ml/min.; detection wavelength: 210, 225, and 254 nm. Retention time of N-benzyl-4-hydroxy-2-pyrrolidinone: 2.7 min.; retention time of N-benzyl-2-pyrrolidinone: 8.1 min.

The crude product was obtained by standard work-up: the cells were removed by centrifugation, the supernatants were adjusted to pH = 8-12 by the addition of KOH followed by extraction with ethyl acetate. The organic phase was dried over MgSO₄, filtered, and the solvent evaporated.

The product was purified by column chromatography on silica gel (R_{f} = 0.14, ethyl acetate/CHCl₃ 9/1) and identified as N-benzyl-4-hydroxy-2-pyrrolidinone by comparing the MS spectra and NMR spectra with those of the authentic compound.

### Example 3: Hydroxylation of N-tert-butoxycarbonyl-2-pyrrolidinone giving N-tert-butoxycarbonyl-4-hydroxy-2'pyrrolidinone with resting cells of Sphingomonas sp. HXN-200

N-tert-Butoxycarbonyl-2-pyrrolidinone (2-18 mM) was added to 10 ml cell suspension (4.0 g/L) of Sphingomonas sp. HXN-200 in 50 mM K-phosphate buffer (pH 8.0) containing glucose (0 or 2%), and the mixture was shaken at 30°C for 5 h. The reaction was followed by analytical HPLC: samples were taken out directly from the reaction mixture at different times, the cells were removed by centrifugation, and the supernatants were analysed. The results are listed in table 3.

**Table 3:**

| Hydroxylation of N-tert-butoxycarbonyl-2-pyrrolidinone giving N-tert-butoxycarbonyl-4-hydroxy-2-pyrrolidinone with resting cells (4.0 g/L) of Sphingomonas sp. HXN-200 | | | | | | | |
|---|---|---|---|---|---|---|---|
| Entry | Substr. (mM) | Activity¹ (U/g CDW) | Conversion (%) | | | | |
| | | | 0.5 h | 1 h | 2 h | 3 h | 5 h |
| 1 | 2 | 4.4 | 26 | 47 | 67 | 73 | 80 |
| 2 | 5 | 6.8 | 16 | 34 | 54 | 66 | 76 |
| 3 | 6 | 7.7 | 15 | 32 | 53 | 65 | 74 |
| 4 | 7 | 8.4 | 14 | 30 | 51 | 63 | 73 |
| 5 | 8 | 8.9 | 13 | 28 | 48 | 61 | 71 |
| 6 | 9 | 8.4 | 11 | 24 | 42 | 55 | 67 |
| 7 | 10 | 8.5 | 10 | 22 | 39 | 52 | 65 |
| 8 | 12 | 9.2 | 9 | 22 | 39 | 51 | 62 |
| 9 | 14 | 10.7 | 9 | 22 | 40 | 51 | 63 |
| 10 | 16 | 9.5 | 7 | 19 | 34 | 45 | 57 |
| 11 | 18 | 9.2 | 6 | 14 | 27 | 36 | 48 |

| | | | | | | | |
|---|---|---|---|---|---|---|---|
| ¹Activity was determined over the first 30 min. | | | | | | | |

Condition of analytical HPLC: column: Hypersil BDS-C18 (5 µm), 125 mm x 4 mm; eluent: acetonitrile/10mM K-phosphate buffer (pH 7.0) 2:8; flow rate: 1 ml/min.; detection wavelength: 210, 225, and 254 nm. Retention time of N-tert-butoxycarbonyl-4-hydroxy-2-pyrrolidinone: 2.7 min.; retention time of N-tert-butoxycarbonyl-2-pyrrolidinone: 6.7 min.

The crude product was obtained by standard work-up: the cells were removed by centrifugation, the supernatants were adjusted to pH = 8-12 by the addition of KOH followed by extraction with ethyl acetate. The organic phase was dried over MgSO₄, filtered, and the solvent evaporated.

The product was purified by column chromatography on silica gel (R_{f} = 0.28, ethyl acetate) and identified as N-tert-butoxycarbonyl-4-hydroxy 2-pyrrolidinone by comparing the MS spectra and NMR spectra with those of the authentic compound.

### Example 4: Preparation of (S)-N-benzyl-4-hydroxy-2-pyrrolidinone by hydroxylation of N-benzyl-2-pyrrolidinone with resting cells of Sphingomonas sp. HXN-200

N-benzyl-2-pyrrolidinone (26.3 mg, 0.15 mmol) was added to 50 ml of cell suspension (4.0 g/L) of Sphingomonas sp. HXN-200 in 50 mM K-phosphate buffer (pH = 8.0) containing glucose (2%) in a 500 ml shaking flask. The mixture was shaken at 200 rpm and 30°C and the bioconversion was followed by HPLC analysis. Samples were taken out at different times (6 x 0.10 ml). The reaction was stopped at 5 h with 66% conversion to N-benzyloxycarbonyl-4-hydroxy-2-pyrrolidinone, as shown in entry 1 of table 4. Standard work-up and column chromatography on silica gel (R_{f} = 0.14, ethyl acetate/CHCl₃ 9:1) afforded 55 % (15.8 mg) of N-benzyl-4-hydroxy-2-pyrrolidinone.
¹H NMR (CDCl₃): δ 7.35-7.19 (m, 5 H, aromatic H), 4.52-4.38 (m, 3 H, NCH₂Ph, H-C(4)), 3.48 (dd, 1 H, J = 10.9 and 5.6 Hz, H_{A}-C(5)), 3.26 (s, br., 1 H, OH), 3.18 (dd, 1, H, J = 10.8Hz, 2.0Hz, H_{B}-C(5)), 2.70 (dd, 1 H, J = 17.4Hz, 6.6Hz, H_{A}-C(3)), 2.43 ppm (dd, 1 H, J = 17.3Hz, 2.5Hz, H_{B}-C(3)).
¹³C NMR (CDCl₃): δ 172.94 (s, CO); 135.97 (s), 128.70 (d), 127.98 (d), 127.60 (d) (aromatic C); 64.27 (d, C-4); 55.71 (t, CH₂Ph); 46.32 (t, C-5); 41.14 ppm (t, C-3).
MS: 192.1(M⁺+1, 100), 174.1(9).

The e.e. of N-benzyl-4-hydroxypyrrolidone was measured by analytical HPLC with a chiral column [Chiralpak AS (Daicel), 250 mm x 4.6 mm; eluent: hexane/isopropanol (4:1); flow rate: 1.0 ml/min.; detection wavelength: 210nm; retention times: 20.3 min. for the (S)-form and 30.5 min. for the (R)-form]. The e.e. of N-benzyl-4-hydroxy 2-pyrrolidinone obtained here is >99.9% (S).

**Table 4:**

| Preparation of (S)-N-benzyl-4-hydroxy 2-pyrrolidinone in >99.9% e.e. by hydroxylation of N-benzyl 2-pyrrolidinone with resting cells of Sphingomonas sp. HXN-200 | | | | | | | | | |
|---|---|---|---|---|---|---|---|---|---|
| Entry | Substr. (mM) | Cell density (g/L) | Conversion (%) | | | | | | Yield (%) |
| | | | 0.5 h | 1 h | 2 h | 3 h | 4 h | 5 h | |
| 1 | 3 | 4.0 | 17 | 28 | 47 | 59 | 63 | 66 | 55 |
| 2 | 6 | 8.0 | 14 | 27 | 41 | 49 | 52 | 51 | 42 |
| 3 | 6 | 10.0 | 11 | 28 | 48 | 63 | 71 | 75 | 68 |

In Entry 2, hydroxylation of 6 mM (52.5 mg) of N-benzyl 2-pyrrolidinone with resting cells (8.0 g/L) of Sphingomonas sp. HXN-200 for 5 h formed 51% of N-benzyl-4-hydroxy-2-pyrrolidinone. Standard work-up and column chromatography gave 42% (23.5 mg) of N-benzyl-4-hydroxy 2-pyrrolidinone in >99.9% (S).

Hydroxylation of 6 mM (52.5 mg) of N-benzyl-2-pyrrolidinone with resting cells (10.0 g/L) of Sphingomonas sp. HXN-200 for 5 h gave 75% conversion to N-benzyl-4-hydroxy-2-pyrrolidinone, as shown in Entry 3. The pure compound was isolated in 68% (39.0 mg) yield with >99.9% (S).

### Example 5: Preparation of (S)-N-tert-butoxycarbonyl-4-hydroxy-2-pyrrolidinone by hydroxylation of N-tert-butoxycarbonyl-2-pyrrolidinone with resting cells of Sphingomonas sp. HXN-200

N-tert-butoxycarbonyl-2-pyrrolidinone (129.5 mg, 0.70 mmol) was added to 50 ml of cell suspension (8.0 g/L) of Sphingomonas sp. HXN-200 in 50 mM K-phosphate buffer (pH 8.0) containing glucose (2%) in a 500 ml shaking flask. The mixture was shaken at 200 rpm and 30°C and the bioconversion was followed by HPLC analysis of samples taken out at different time (6 x 0.10 ml). The reaction was stopped at 5 h with 66% conversion to N-tert-butoxycarbonyl-4-hydroxy-2-pyrrolidinone. Standard work-up and column chromatography on silica gel (R_{f} = 0.28, ethyl acetate) afforded 46% (63.3 mg) of the pure product. The result was shown in Entry 2 of Table 5.
¹H NMR (CDCl₃): 4.47 (s, 1 H, H-C(4)), 3.88 (dd, 1 H, J = 11.9Hz, 5.1Hz, H_{A}-C(5)), 3.77 (d, 1 H, J = 11.8Hz, H_{B}-C(5)), 3.15 (s, 1 H, OH), 2.77 (dd, 1 H, J = 17.7 and 6.1Hz, H_{A}-C(3)), 2.43 (d, 1 H, J = 17.7Hz, H_{B}-C(3)), 1.52 ppm (s, 9 H, 3CH₃).
¹³C NMR (CDCl₃): δ 172.8 (s, COO); 150.05 (s; CO); 83.19 (s, C(CH₃)₃); 63.03 (d, C-4); 55.31 (t, C-5); 42.71 (t, C-3); 28.03 ppm (q, CH₃).
MS: 202 (M⁺+1, 2), 146.0 (100), 128.0 (13), 113.0 (12), 102.1 (81).

The e.e. of N-tert-butoxycarbonyl-4-hydroxy-2-pyrrolidinone was measured by analytical HPLC with a chiral column [Chiralcel OB-H (Daicel), 250 mm x 4.6 mm; eluent: hexane/isopropanol (7:1); flow rate: 0.5 ml/min.; detection wavelength: 210 nm; retention times: 17.9 min. for the (R)-form and 22.6 min. for the (S)-form]. The product has 92% e.e. (S).

**Table 5:**

| Preparation of (S)-N-tert-butoxycarbonyl-4-hydroxy-2-pyrrolidinone by hydroxylation of N-tert-butoxycarbonyl-2-pyrrolidinone with resting cells of Sphingomonas sp. HXN-200 | | | | | | | | | | |
|---|---|---|---|---|---|---|---|---|---|---|
| Entry | Substr. (mM) | Cell density (g/L) | Conversion (%) | | | | | | Yield (%) | e.e. (S) (%) |
| | | | 0.5 h | 1 h | 2 h | 3 h | 4 h | 5 h | | |
| 1 | 14 | 4.0 | 6 | 12 | 25 | 35 | 43 | 48 | 39 | 90 |
| 2 | 14 | 8.0 | 10 | 20 | 38 | 52 | 61 | 66 | 46 | 92 |
| 3 | 20 | 10 | 8 | 17 | 35 | 41 | 45 | 49 | 42 | 90 |

Hydroxylation of 14 mM (129.5 mg) of N-tert-butoxycarbonyl-2-pyrrolidinone with resting cells (4.0 g/L) of Sphingomonas sp. HXN-200 for 5 h gave 48% conversion to N-tert-butoxycarbonyl-4-hydroxy-2-pyrrolidinone. The pure product was obtained in 39% (54.6 mg) yield with 90% e.e. (S). The results are summarized in Entry 1 of table 5.

Hydroxylation of 20 mM (185.0 mg) of N-tert-butoxycarbonyl-2-pyrrolidinone with resting cells (10.0 g/L) of HXN-200 for 5 h formed 49% of N-tert-butoxycarbonyl-4-hydroxy-2-pyrrolidinone that was isolated in 42% (84.2 mg) yield with 90 % e.e. (S), as shown in Entry 3 of table 5.

### Increase of e.e. of (S)-N-tert-butoxycarbonyl-4-hydroxy-2-pyrrolidinone by crystallization

The biohydroxylation product (S)-N-tert-butoxycarbonyl-4-hydroxy-2-pyrrolidinone (92% e.e., 26.3 mg) was dissolved in 1.5ml of n-hexane/ethyl acetate (2/1) and crystallized at 0°C for 24 h. Filtration afforded 21.6 mg (82%) of the crystals of pure product in 99.9% e.e. (S).

### Example 6: Preparation of (S)-N-benzyl-4-hydroxy-2-pyrrolidinone by hydroxylation of N-benzyl-2-pyrrolidinone with resting, cells (10.0 g/L) of Sphingomonas sp. HXN-200 in a bioreactor

N-Benzyl-2-pyrrolidinone (1.852 g, 10.0 mmol) was added to a cell suspension (10.0 g/L) of Sphingomonas sp. HXN-200 in 1 L of 50 mM of K-phosphate buffer (pH = 8.0) containing glucose (2%, w/v) in a 3 L bioreactor, the mixture was stirred at 1500 rpm and at 30°C under the introduction of air at 2 L/min. The biotransformation was stopped at 5 h with 80% conversion to N-benzyl-4-hydroxy-2-pyrrolidinone. Standard work-up and column chromatography on silica gel (R_{f} = 0.14, ethyl acetate/CHCl₃ 9:1) gave 1.408 g (70%) of pure product as white powder in >99.9% e.e. (S).

### Example 7: Preparation of (S)-N-tert-butoxycarbonyl-4-hydroxy-2-pyrrolidinone by hydroxylation of N-tert-butoxycarbonyl-2-pyrrolidinone with resting cells (10.2 g) of Sphingomonas sp. HXN-200 in a bioreactor

N-tert-butoxycarbonyl-2-pyrrolidinone (2.453 g, 10 mmol) was added to a cell suspension (10.2 g/L) of Sphingomonas sp. HXN-200 in 1 L of 50 mM of K-phosphate buffer (pH = 8.0) containing glucose (2%, w/v) in a 3 L bioreactor, the mixture was stirred at 1500 rpm and at 30°C under the introduction of air at 2 L/min. The biotransformation was stopped at 5 h with 85% conversion to N-tert-butoxycarbonyl-4-hydroxy-2-pyrrolidinone. Standard work-up and column chromatography on silica gel (R_{f} = 0.28, ethyl acetate) gave 2.008 g (75%) of pure product as white powder in 90% e.e. (S).

### Example 8: Preparation of (S)-N-benzyl-4-hydroxy-2-pyrrolidinone by hydroxylation of N-benzyl-2-pyrrolidinone with growing cells of Sphingomonas sp. HXN-200 in a bioreactor

The cells of Sphingomonas sp. HXN-200 were grown in 1 L E2 medium first on glucose and then on n-octane to a cell density of 6.2 g/L. N-benzyl-2-pyrrolidinone (0.741 g, 4.0 mmol) was added; and the mixture was stirred at 1536 rpm and at 30°C with air introduction at 2 L/min. During bioconversion, n-octane vapour was still introduced and the cells were still grown. Additional substrate was added at 1 h (0.370 g, 2.0 mmol), 2 h (0.370 g, 2.0 mmol), and 3 h (0.370 g, 2.0 mmol). The reaction was followed by analytical HPLC and stopped at 4 h with 84% conversion to N-benzyl-4-hydroxy-2-pyrrolidinone. Standard work-up and column chromatography afforded 1.529 g (76%) of pure product in >99.9% e.e. (S).

### Example 9: Preparation of (S)-N-benzyl-4-hydroxy-2-pyrrolidinone by hydroxylation of N-benzyl-2-pyrrolidinone with cell-free extract of Sphingomonas sp. HXN-200

Cells of HXN-200 were suspended in 10 ml of Tris-HCl buffer (pH = 7.5) to a density of 20 g/L. After passage through the French press three times, the cell debris was removed by centrifugation at 45000 rpm (Rotor Type 50.2 Ti) for 45 min. yielding soluble cell-free extracts containing no membrane proteins. To this cell-free extracts was added NADH (40 µl of 500 mM aqueous solution, 0.02 mmol) and N-benzyl-2-pyrrolidinone (3.7 mg, 0.02 mmol). The mixture was shaken at 200 rpm and at 30°C for 1 h, and afforded 80% of N-benzyl-4-hydroxy-2-pyrrolidinone in >99.9% e.e. (S).

## Claims

1. A process for the preparation of optically active 4-hydroxy-2-pyrrolidinone or an N-substituted 4-hydroxy-2-pyrrolidinone by hydroxylation of a corresponding non-hydroxylated 2-pyrrolidinone or N-substituted 2-pyrrolidinone using as a biocatalyst alkane-degrading bacteria having hydroxylation activity to insert an oxygen atom regio- and stereoselectively into said corresponding non-hydroxylated 2-pyrrolidinone or N-substituted 2-pyrrolidinone.

2. The process of claim 1, wherein the alkane-degrading bacterium is selected from the group consisting of bacteria that degrade alkanes containing 4 to 20 carbon atoms.

3. The process of claim 2, wherein the alkane-degrading bacterium is selected from the group consisting of bacteria that degrade n-hexane, n-heptane, n-octane, n-nonane, n-decane, n-undecane, n-dodecane, n-tridecane, or n-tetradecane.

4. The process of claim 1, wherein the alkane-degrading bacterium is selected from the group consisting of bacteria that degrade cyclic compounds containing 4 to 20 carbon atoms.

5. The process of claim 4, wherein the alkane-degrading bacterium selected from the group consisting of bacteria that degrade cyclopentane, cyclohexane, cycloheptane, cyclooctane, cyclononane, cyclodecane, cycloundecane, cyclododecane, cyclotridecane, or cyclotetradecane.

6. The process of claim 1, wherein resting cells of bacteria growing cells of bacteria, or both, are used as the biocatalyst.

7. The process of claim 1, wherein a crude cell extract is used as the biocatalyst.

8. The process of claim 1, wherein the biocatalyst is immobilized on or in a water-insoluble carrier or support system.

9. The process of claim 1, wherein the biocatalytic reaction is performed in aqueous medium.

10. The process of claim 1, wherein the biocatalytic reaction is performed in multiphase media containing two or more of the following: a solid phase, an aqueous phase, an organic phase, and a gaseous phase.

11. The process of claim 10, wherein an organic phase is used which comprises one or more of the following: alkanes with 5 or more C atoms; dialkyl ethers with 4 or more C atoms; carboxylic esters with 3 or more C atoms; aromatic or heteroaromatic hydrocarbons with 6 or more C atoms; or alkyl aromatic or alkyl heteroaromatic hydrocarbons with 7 or more C atoms.

12. The process of claim 1, wherein the reaction temperature is 5-50°C, preferably 20-40°C.

13. The process of claim 1, wherein the pH of the medium is 4-10, preferably 20 6-8.

14. The process of claim 1, wherein the product is separated by means of a chromatographic technique with an inorganic, organic or synthetic adsorbent used as a support.

15. The process of claim 1, wherein the product is separated by means of extraction, wherein the substrate is first recovered from the reaction mixture by extraction with a first solvent, the remaining reaction mixture is adjusted to pH = 8-12, and the product is extracted out with a second solvent, wherein said second solvent is more polar compared to said first solvent.

16. The process of claim 15, wherein either one of the extraction agents used, or both, are selected from the group consisting of: alkanes with 5 or more C atoms; dialkyl ethers with 4 or more C atoms; chlorine-containing alkanes with 3 or fewer C atoms; carboxylic esters with 3 or more C atoms; aromatic or heteroaromatic hydrocarbons with 6 or more C atoms; and alkyl aromatic or alkyl heteroaromatic hydrocarbons with 7 or more C atoms.

17. The process of claim 1, wherein the product is separated by means of solid phase extraction.

18. The process of claim 1, wherein the product is separated by use of membrane filtration.

19. The process of claim 1, wherein the said N-substituted 4-hydroxy-2-pyrrolidinone is N-benzyl-4-hydroxy-2-pyrrolidinone.

20. The process of claim 1, wherein the said N-substituted 4-hydroxy-2-pyrrolidinone is N-tert-butoxycarbonyl-4-hydroxy-2-pyrrolidinone.

21. The process of claim 1, wherein the said N-substituted 4-hydroxy-2-pyrrolidinone is N-benzoyl-4-hydroxy-2-pyrrolidinone.

22. The process of claim 1, wherein the said N-substituted 4-hydroxy-2-pyrrolidinone is N-benzyloxycarbonyl-4-hydroxy-2-pyrrolidinone.

23. The process of claim 1, wherein the said N-substituted 4-hydroxy-2-pyrrolidinone is N-phenoxycarbonyl-4-hydroxy-2-pyrrolidinone.

24. The process of claim 1, wherein the said N-substituted 4-hydroxy-2-pyrrolidinone is N-tosyl-4-hydroxy-2-pyrrolidinone.

## Patentansprüche

1. Verfahren zur Herstellung eines optisch aktiven 4-Hydroxy-2-pyrrolidinons oder eines N-substituierten 4-Hydroxy-2-pyrrolidons durch Hydroxylierung eines korrespondierenden nicht hydroxylierten 2-Pyrrolidinons oder N-substituierten 2-Pyrrolidinons unter Verwendung von Alkan abbauenden Bakterien mit hydroxylierender Aktivität als Biokatalysatoren, um regio- und stereoselektiv ein Sauerstoffatom in das korrespondierende nicht hydroxylierte 2-Pyrrolidinon oder N-substituierte 2-Pyrrolidinon einzubringen.

2. Verfahren nach Anspruch 1, worin das Alkan abbauende Bakterium aus der Gruppe ausgewählt ist, die aus Bakterien besteht, die Alkane, die 4 bis 20 Kohlenstoffatome enthalten, abbauen.

3. Verfahren nach Anspruch 2, worin das Alkan abbauende Bakterium aus der Gruppe ausgewählt ist, die aus Bakterien besteht, die n-Hexan, n-Heptan, n-Octan, n-Nonan, n-Decan, n-Undecan, n-Dodecan, n-Tridecan, oder n-Tetradecan abbauen.

4. Verfahren nach Anspruch 1, worin das Alkan abbauende Bakterium aus der Gruppe ausgewählt ist, die aus Bakterien besteht, die zyklische 4 bis 20 Kohlenstoffatome enthaltende Verbindungen abbauen.

5. Verfahren nach Anspruch 4, worin das Alkan abbauende Bakterium aus der Gruppe ausgewählt ist, die aus Bakterien besteht, die Cyclopentan, Cyclohexan, Cycloheptan, Cyclooctan, Cyclononan, Cyclodecan, Cycloundecan, Cyclododecan, Cyclotridecan oder Cyclotetradecan abbauen.

6. Verfahren nach Anspruch 1, worin ruhende Bakterienzellen, wachsende Bakterienzellen oder beide als Biokatalysator verwendet werden.

7. Verfahren nach Anspruch 1, worin ein roher Zellextrakt als Biokatalysator verwendet wird.

8. Verfahren nach Anspruch 1, worin der Biokatalysator auf oder in einem wasserunlösslichen Träger oder Trägersystem immobilisiert ist.

9. Verfahren nach Anspruch 1, worin die biokatalytische Reaktion in wässerigem Medium durchgeführt wird.

10. Verfahren nach Anspruch 1, worin die biokatalytische Reaktion in mehrphasigen Medien durchgeführt wird, welche zwei oder mehrere der folgenden Phasen enthält: eine feste Phase, eine wässerige Phase, eine organische Phase und eine gasförmige Phase.

11. Verfahren nach Anspruch 10, worin eine organische Phase verwendet wird, welche zwei oder mehrere der folgenden Verbindungen umfasst: Alkane mit 5 oder mehr C-Atomen; Dialkylether mit 4 oder mehr C-Atomen; Carbonsäureester mit 3 oder mehr C Atomen; aromatische oder heteroaromatische Kohlenwasserstoffe mit 6 oder mehr C-Atomen; oder alkylaromatische oder alkyl-heteroaromatische Kohlenwasserstoffe mit 7 oder mehr C-Atomen.

12. Verfahren nach Anspruch 1, worin die Reaktionstemperatur 5 bis 50 °C, vorzugsweise 20 bis 40 °C ist.

13. Verfahren nach Anspruch 1, worin der pH-Wert des Mediums 4 bis 10, vorzugsweise 6 bis 8 ist.

14. Verfahren nach Anspruch 1, worin das Produkt mittels einer chromatographischen Technik unter Verwendung eines anorganischen, organischen oder synthetischen Adsorptionsmittels als Träger abgetrennt wird.

15. Verfahren nach Anspruch 1, worin das Produkt mittels Extraktion abgetrennt wird, worin das Substrat zuerst durch Extraktion mit einem ersten Lösungsmittel aus dem Reaktionsgemisch entfernt, das verbleibende Reaktionsgemisch auf einen pH = 8 - 12 eingestellt und das Produkt mit einem zweiten Lösungsmittel extrahiert wird, wobei das zweite Lösungsmittel, verglichen mit dem ersten Lösungsmittel, polarer ist.

16. Verfahren nach Anspruch 15, worin jeweils eine der verwendeten Extraktionsmittel oder beide aus der Gruppe, bestehend aus Alkanen mit 5 oder mehr C-Atomen; Dialkylethern mit 4 oder mehr C-Atomen; chlorhaltigen Alkanen mit 3 oder weniger C-Atomen; Carbonsäureestern mit 3 oder mehr C-Atomen; aromatischen oder heteroaromatischen Kohlenwasserstoffen mit 6 oder mehr C-Atomen; und alkyl-aromatischen oder alkyl-heteroaromatischen Kohlenwasserstoffen mit 7 oder mehr C-Atomen ausgewählt sind.

17. Verfahren nach Anspruch 1, worin das Produkt mittels Festphasenextraktion abgetrennt wird.

18. Verfahren nach Anspruch 1, worin das Produkt mittels Membranfiltration abgetrennt wird.

19. Verfahren nach Anspruch 1, worin das N-substituierte 4-Hydroxy-2-pyrrolidinon N-Benzyl-4-hydroxy-2-pyrrolidinon ist.

20. Verfahren nach Anspruch 1, worin das N-substituierte 4-Hydroxy-2-pyrrolidinon N*-tert*-Butoxycarbonyl-4-hydroxy-2-pyrrolidinon ist.

21. Verfahren nach Anspruch 1, worin das N-substituierte 4-Hydroxy-2-pyrrolidinon N-Benzoyl-4-hydroxy-2-pyrrolidinon ist.

22. Verfahren nach Anspruch 1, worin das N-substituierte 4-Hydroxy-2-pyrrolidinon N-Benzyloxycarbonyl-4-hydroxy-2-pyrrolidinon ist.

23. Verfahren nach Anspruch 1, worin das N-substituierte 4-Hydroxy-2-pyrrolidinon N-Phenoxycarbonyl-4-hydroxy-2-pyrrolidinon ist.

24. Verfahren nach Anspruch 1, worin das N-substituierte 4-Hydroxy-2-pyrrolidinon N-tosyl-4-Hydroxy-2-pyrrolidinon ist.

## Revendications

1. Procédé pour la préparation de 4-hydroxy-2-pyrrolidinone optiquement active ou d'une 4-hydroxy-2-pyrrolidinone N-substituée par hydroxylation d'une 2-pyrrolidinone non hydroxylée ou d'une 2-pyrrolidinone N-substituée correspondante, utilisant, en tant que biocatalyseur, des bactéries dégradant les alcanes ayant une activité d'hydroxylation pour insérer une régio- et une stéréo-sélectivité au niveau de l'atome d'oxygène dans ladite 2-pyrrolidinone non-hydroxylée ou 2-pyrrolidinone N-substituée correspondante.

2. Procédé selon la revendication 1, dans lequel la bactérie dégradant les alcanes est choisie dans le groupe constitué par les bactéries qui dégradent les alcanes contenant de 4 à 20 atomes de carbone.

3. Procédé selon la revendication 2, dans lequel la bactérie dégradant les alcanes est choisie dans le groupe constitué par les bactéries qui dégradent le n-hexane, le n-heptane, le n-octane, le n-nonane, le n-décane, le n-undécane, le n-dodécane, le n-tridécane, ou le n-tétradécane.

4. Procédé selon la revendication 1, dans lequel la bactérie dégradant les alcanes est choisie dans le groupe constitué par les bactéries qui dégradent les composés cycliques contenant de 4 à 20 atomes de carbone.

5. Procédé selon la revendication 4, dans lequel la bactérie dégradant les alcanes est choisie dans le groupe constitué par les bactéries qui dégradent le cyclopentane, le cyclohexane, le cycloheptane, le cyclooctane, le cyclononane, le cyclodécane, le cycloundécane, le cyclododécane, le cyclotridécane, ou le cyclotétradécane.

6. Procédé selon la revendication 1, dans lequel on utilise, en tant que biocatalyseur, des cellules au repos de bactéries, des cellules en croissance de bactéries, ou les deux.

7. Procédé selon la revendication 1, dans lequel un extrait cellulaire brut est utilisé en tant que biocatalyseur.

8. Procédé selon la revendication 1, dans lequel le biocatalyseur est immobilisé sur ou dans un véhicule ou un système de support insoluble dans l'eau.

9. Procédé selon la revendication 1, dans lequel la réaction biocatalytique est effectuée dans un milieu aqueux.

10. Procédé selon la revendication 1, dans lequel la réaction biocatalytique est effectuée dans un milieu à phases multiples contenant deux ou plus des suivantes : une phase solide, une phase aqueuse, une phase organique, et une phase gazeuse.

11. Procédé selon la revendication 10, dans lequel on utilise une phase organique qui comprend un ou plusieurs des suivants : des alcanes ayant 5 atomes C ou plus ; des dialkyléthers ayant 4 atomes C ou plus ; des esters carboxylates ayant 3 atomes C ou plus ; des hydrocarbures aromatiques ou hétéroatomatiques ayant 6 atomes C ou plus ; ou des hydrocarbures alkylaromatiques ou alkylhétéroaromatiques ayant 7 atomes C ou plus.

12. Procédé selon la revendication 1, dans lequel la température de réaction est de 5 à 50°C, de préférence de 20 à 40°C.

13. Procédé selon la revendication 1, dans lequel le pH du milieu est de 4 à 10, de préférence de 6 à 8.

14. Procédé selon la revendication 1, dans lequel le produit est séparé au moyen d'une technique chromatographique avec un adsorbant inorganique, organique ou synthétique utilisé en tant que support.

15. Procédé selon la revendication 1, dans lequel le produit est séparé au moyen d'une extraction où le substrat est d'abord récupéré à partir du mélange réactionnel par extraction avec un premier solvant, le mélange réactionnel restant est ajusté à un pH de 8 à 12, et le produit est extrait avec un deuxième solvant, où ledit deuxième solvant est davantage polaire que ledit premier solvant.

16. Procédé selon la revendication 15, dans lequel l'un des agents d'extraction utilisés, ou les deux, sont choisis dans le groupe constitué par : les alcanes ayant 5 atomes C ou plus ; les dialkyléthers ayant 4 atomes C ou plus ; les alcanes chlorés ayant 3 atomes C ou moins ; les esters carboxylates ayant 3 atomes C ou plus ; les hydrocarbures aromatiques ou hétéroaromatiques ayant 6 atomes C ou plus ; et les hydrocarbures alkylaromatiques ou alkylhétéroaromatiques ayant 7 atomes C ou plus.

17. Procédé selon la revendication 1, dans lequel le produit est séparé au moyen d'une extraction en phase solide.

18. Procédé selon la revendication 1, dans lequel le produit est séparé par utilisation d'une filtration à membrane.

19. Procédé selon la revendication 1, dans lequel ladite 4-hydroxy-2-pyrrolidinone N-substituée est la N-benzyl-4-hydroxy-2-pyrrolidinone.

20. Procédé selon la revendication 1, dans lequel ladite 4-hydroxy-2-pyrrolidinone N-substituée est la N-tert-butoxycarbonyl-4-hydroxy-2-pyrrolidinone.

21. Procédé selon la revendication 1, dans lequel ladite 4-hydroxy-2-pyrrolidinone N-substituée est la N-benzyl-4-hydroxy-2-pyrrolidinone.

22. Procédé selon la revendication 1, dans lequel ladite 4-hydroxy-2-pyrrolidinone N-substituée est la N-benzyloxycarbonyl-4-hydroxy-2-pyrrolidinone.

23. Procédé selon la revendication 1, dans lequel ladite 4-hydroxy-2-pyrrolidinone N-substituée est la N-phénoxycarbonyl-4-hydroxy-2-pyrrolidinone.

24. Procédé selon la revendication 1, dans lequel ladite 4-hydroxy-2-pyrrolidinone N-substituée est la N-tosyl-4-hydroxy-2-pyrrolidinone.
